(19) Europäisches Patentamt
European Patent Office
Office européen des brevets

(11) EP 4 064 843 B1

(12) # EUROPEAN PATENT SPECIFICATION

(45) Date of publication and mention of the grant of the patent:
**18.02.2026 Bulletin 2026/08**

(21) Application number: **20811348.0**

(22) Date of filing: **26.11.2020**

(51) International Patent Classification (IPC):
***A01P 1/00*** *(2006.01)* ***A01N 35/02*** *(2006.01)*

(52) Cooperative Patent Classification (CPC):
**A01N 35/02**

(86) International application number:
**PCT/EP2020/083516**

(87) International publication number:
**WO 2021/105288 (03.06.2021 Gazette 2021/22)**

(54) **USE OF A COMPOUND OF 4-(3-ETHOXY-4-HYDROXYPHENYL) ALKYL KETONE TYPE AGAINST BACTERIA OF THE BURKHOLDERIA CEPACIA COMPLEX**

VERWENDUNG VON VERBINDUNGEN DES 4-(3-ETHOXY-4-HYDROXYPHENYL) ALKYLKETONTYPS GEGEN BACTERIEN VOM BURKHOLDERIA CEPACIA KOMPLEX

UTILISATION D'UN COMPOSÉ DE TYPE 4-(3-ÉTHOXY-4 HYDROXYPHÉNYL) ALKYLCÉTONE CONTRE LES BACTÉRIES DU COMPLEXE BURKHOLDERIA CEPACIA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2019 FR 1913366**

(43) Date of publication of application:
**05.10.2022 Bulletin 2022/40**

(73) Proprietor: **L'OREAL**
**75008 Paris (FR)**

(72) Inventors:
- **CUPFERMAN, Sylvie**
  **94152 CHEVILLY LA RUE (FR)**
- **MENARD-SZCZEBARA, Florence**
  **94152 CHEVILLY LA RUE (FR)**

(74) Representative: **L'Oreal**
**Service D.I.P.I.**
**9, rue Pierre Dreyfus**
**92110 Clichy (FR)**

(56) References cited:
**FR-A1- 3 073 397**

- **DINESH RAGHAVAN ET AL: "Isolation, characterization, and evaluation of multi-trait plant growth promoting rhizobacteria for their growth promoting and disease suppressing effects on ginger", MICROBIOLOGICAL RESEARCH, vol. 173, 11 February 2015 (2015-02-11), pages 34 - 43, XP029149370, ISSN: 0944-5013, DOI: 10.1016/J.MICRES.2015.01.014**
- **STARTEK JUSTYNA B ET AL: "To flourish or perish: evolutionary TRiPs into the sensory biology of plant-herbivore interactions", PFLUEGERS ARCHIV: EUROPEAN JOURNAL OF PHYSIOLOGY, SPRINGER VERLAG, BERLIN, DE, vol. 471, no. 2, 18 September 2018 (2018-09-18), pages 213 - 236, XP036678358, ISSN: 0031-6768, [retrieved on 20180918], DOI: 10.1007/S00424-018-2205-1**
- **LAURA RUSHTON ET AL: "ABSTRACT", ANTIMICROBIAL AGENTS AND CHEMOTHERAPY, vol. 57, no. 7, 1 July 2013 (2013-07-01), US, pages 2972 - 2980, XP055700990, ISSN: 0066-4804, DOI: 10.1128/AAC.00140-13**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 064 843 B1

## Description

The present invention relates to the use of a compound of 4-(3-ethoxy-4-hydroxyphenyl) alkyl ketone type in the treatment of products or compositions which are aqueous or nonaqueous, preferably water and aqueous compositions, against bacteria of the *Burkholderia cepacia* complex (Bcc), and also the use thereof in a treatment process with respect to strains of the Bcc complex.

Antimicrobial agents are widely used in healthcare establishments, dwellings, farms and industries, for inhibiting the proliferation of damaging organisms and killing potential pathogens.

Microbial contamination during the production of an industrial product is common, even when starting ingredients placed in said product are "clean", i.e. no contaminating microorganisms are present. Water, in particular, which is omnipresent in most cosmetic, pharmaceutical or food products, must be free of contaminating microorganisms. The cleanliness during production of these industrial products, the processing of the contents and the filling of the containers must be scrupulously monitored, as must the industrial equipment. Despite these precautions, the microbial integrity of the products generally requires the presence of one or more preservatives compatible with the product and the stability of the composition. The products must allow neither growth nor survival of contaminating microorganisms. Even if it is industrially possible to carry out a restrictive production in a sterile environment, maintaining the stability during use is problematic, since fingers, cosmetic applicators and even ambient air are not sterile. Preservatives are therefore required in order to reduce contamination with microorganisms by consumers during normal use. As a general rule, pathogenic microorganisms must be absent from all products sold, in particular cosmetic products (Kirk Othmer Encyclopedia, Cosmetics, Martin M. Rieger, 04/12/2000; https://doi.org/10.1002/0471238961.0315191318090507.a01 and standard ISO 17516 Cosmetics - Microbiology - Microbiological limits).

In industry, antimicrobial combinations are incorporated at low concentration in finished products and starting materials as preservatives for protecting against microbial contamination. The failure of these preservation systems can lead to a considerable economic loss and, depending on the contaminant, can present a risk to the health of the consumer. A wide range of yeasts, molds, fungi and bacteria may be responsible for product contaminations. Gram-negative bacteria can be encountered in industries, in particular pharmaceutical industries and cosmetic industries, with a predominance in product recalls concerning in particular bacteria of the *Burkholderia cepacia* complex (Jimenez, L., Microbial diversity in pharmaceutical product recalls and environments. PDA Journal of Pharmaceutical Science and Technology, 2007. 61(5): p. 383-399, Rushton, L. Rapid Alert System Weekly Notification Reports. 2005-2014).

Industry is seeking to formulate products comprising robust preservation systems with a broad antimicrobial spectrum, which prevent microbial growth and which do not result in the development of resistant microorganisms. This challenge becomes more complex because of restrictions imposed on the use of preservatives and the pressure exerted by consumers for a milder preservation of products, in particular cosmetic products. Over the past few years, safety problems linked to potential estrogenic activity and to skin sensitization problems have led to a significant reduction in the use of preservatives such as isothiazolinone, which are highly effective agents against bacteria of the Bcc complex (Rushton, L., et al., Key role for efflux in the preservative susceptibility and adaptive resistance of Burkholderia cepacia complex bacteria. Antimicrobial Agents and Chemotherapy, 2013. 57(7): p. 2972-2980). This underlines the need for new preservatives that are highly effective against the key risk species, such as the bacteria of the Bcc complex.

The *Burkholderia cepacia* complex (Bcc) comprises more than 20 closely related but genetically distinct species within the *Burkholderia* genus. As highly adaptable natural bacteria, Bccs have been studied for their potential biotechnological applications in plant promotion, bioremediation and biological control of plant pests (Mahenthiralingam, E., A. Baldwin, and C.G. Dowson, Burkholderia cepacia complex bacteria: Opportunistic pathogens with important natural biology. Journal of Applied Microbiology, 2008. 104(6): p. 1539-1551).

Bccs had been widely studied as opportunistic pathogens that can cause an infection in several hosts, such as chronic respiratory infections in individuals suffering from cystic fibrosis (CF) (Depoorter, E., et al., Burkholderia: an update on taxonomy and biotechnological potential as antibiotic producers. Applied Microbiology and Biotechnology, 2016. 100(12): p. 5215-5229). As industrial contaminants, Bcc bacteria have been isolated from petroleum products (White, J., et al., Culture-independent analysis of bacterial fuel contamination provides insight into the level of concordance with the standard industry practice of aerobic cultivation. Applied and Environmental Microbiology, 2011. 77(13): p. 4527-4538), (Álvarez-Lerma, F., et al., Moisturizing body milk as a reservoir of Burkholderia cepacia: Outbreak of nosocomial infection in a multidisciplinary intensive care unit. Critical Care, 2008. 12(1)), from pharmaceutical products and also cosmetic products, and also from toiletry products (Jimenez, L., Microbial diversity in pharmaceutical product recalls and environments. PDA Journal of Pharmaceutical Science and Technology, 2007. 61(5): p. 383-399). Bcc infection epidemics in vulnerable individuals, although rare, are the result of the use of contaminated industrial products [Álvarez-Lerma, F., et al., Moisturizing body milk as a reservoir of Burkholderia cepacia: Outbreak of nosocomial infection in a multidisciplinary intensive care unit. Critical Care, 2008. 12(1)., Kutty, P.K., et al., Multistate outbreak of Burkholderia cenocepacia colonization and infection associated with the use of intrinsically contaminated alcohol-free mouthwash. Chest, 2007. 132(6): p. 1825-1831], and these bacteria are now recognized as risk species in microbial contamination (Torbeck, L., et

al., Burkholderia cepacia: This decision is overdue. PDA Journal of Pharmaceutical Science and Technology, 2011. 65(5): p. 535-543).

The ability of Bcc bacteria to survive as contaminants is partly due to a high innate antimicrobial resistance and to the ability to adapt to their environment (Mahenthiralingam, E., A. Baldwin, and C.G. Dowson, Burkholderia cepacia complex bacteria: Opportunistic pathogens with important natural biology. Journal of Applied Microbiology, 2008. 104(6): p. 1539-1551). A recent study of Bcc sensitivity to the main preservative groups used in industry has revealed differences in sensitivity between species, for example the levels of sodium benzoate and of benzethonium chloride used appear to be ineffective with respect to contamination by the bacteria of the Bcc complex [Rushton, L., et al., Key role for efflux in the preservative susceptibility and adaptive resistance of Burkholderia cepacia complex bacteria. Antimicrobial Agents and Chemotherapy, 2013. 57(7): p. 2972-2980]. In addition, isolated Bcc strains originating from industrial sources have shown an increased tolerance to a formaldehyde-releasing agent, suggesting Bcc emergence in industry, which is problematic (Rushton, L., et al., *ibid*). These intrinsically resistant opportunistic pathogens are responsible for or contribute to the contamination of industrial products.

Moreover, it is known that water is a vector for the propagation of pathogenic microorganisms; thus, contaminated waters can be responsible for numerous diseases.

Among the pathogenic microorganisms found in aqueous compositions and in water are the bacteria of the *Bcc* complex. These opportunistic bacteria therefore encompass a collection of aerobic Gram -negative pathogenic bacteria, most of which are opportunistic pathogens for human beings, animals and plants.

For public health reasons, there thus remains a real need to provide new active agents that are effective in the treatment of products that may be contaminated by bacteria of the *Bcc* complex, such as food, water and aqueous and anhydrous compositions. In particular, there remains a real need to find new active agents that make it possible i) either to prevent the contamination of industrial or domestic products, in particular water or aqueous or anhydrous compositions, by one or more microorganisms, including bacteria of the *Burkholderia* genus, ii) or to treat consumed products, in particular water or aqueous or anhydrous compositions, contaminated by one or more microorganisms, including bacteria of the *Bcc* complex. Said industrial or domestic products and particularly the water included in aqueous compositions for domestic or industrial uses, in particular products originating from industrial and domestic tanks, waters from aquatic media, swimming pool/spa waters, and air-conditioning system waters, and cosmetic, pharmaceutical and food products.

The object of the present invention is to satisfy these needs.

Surprisingly, the inventors have discovered that the use of at least one compound of formula (I) as defined below, in particular 4-(3-ethoxy-4-hydroxyphenyl) alkyl ketone or ethylzingerone (EZ), makes it possible to reduce the growth of bacteria of the *Burkholderia cepacia* complex of contaminated products. The use of at least one compound of formula (I) as defined below, in particular 4-(3-ethoxy-4-hydroxyphenyl) alkyl ketone or ethylzingerone (EZ), makes it possible to prevent the contamination, by bacteria of the *Burkholderia cepacia* complex, of products that may be in contact with one or more microorganisms, including bacteria of the *Burkholderia* genus, of the *Bcc* complex. Indeed, it appears that the compounds of formula (I) act as a preservative with a high activity against bacteria of the *Burkholderia cepacia* complex leading in particular to a reduction in viability of several log after several hours (6 log in 4 hours).

The compounds of formula (I), in particular EZ, are new preservatives which unexpectedly exert an acute activity against *Burkholderia,* based on a new multifactorial action mode and, furthermore, do not promote resistance to these resilient Gram-negative bacteria, on products that may be contaminated by bacteria of the *Bcc* complex, such as water, industrial or domestic aqueous compositions, industrial or domestic equipment, such as industrial or domestic preparation tanks, the solvents used in industrial or domestic preparations or in industrial equipment, or even industrial or domestic anhydrous compositions.

In addition to the treatment of products that may be contaminated by bacteria of the *Bcc* complex, the use of compounds of formula **(I)** also makes it possible to treat the biofilm present at the surface of devices in contact with water, such as for example water pipes, or at the surface of industrial or domestic tanks.

A subject of the invention is also a process for treating, in particular for preserving, a product that may be contaminated by bacteria of the *Burkholderia cepacia* complex, in particular a composition, comprising particularly a physiologically acceptable medium, more particularly a cosmetic or pharmaceutical composition, characterized in that it consists in incorporating into said product, during the production thereof, or after the production thereof and preferably before storage, one or more compounds of formula (I), preferably EZ.

Thus, the present invention relates to the use of at least one compound of formula (I) and also the organic or mineral acid or base salts thereof, or solvates thereof such as hydrates:

[Chem. 1]

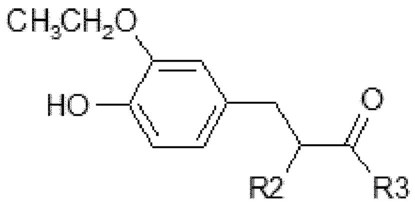

(I)

wherein:

- R2 represents a hydrogen atom or a methyl or ethyl radical;
- R3 represents a linear $C_1$-$C_{12}$ alkyl radical (saturated), optionally substituted with a hydroxyl group; or a linear $C_2$-$C_{12}$ alkenyl radical (C=C unsaturated), optionally substituted with a hydroxyl group;

against bacteria of the *Burkholderia cepacia* complex, in particular in the treatment of products that may be contaminated by bacteria of the *Bcc* complex, such as water, aqueous compositions, anhydrous compositions, equipment, that may be industrial or domestic, in particular for food, cosmetic or pharmaceutical purposes, the solvents used in industrial or domestic preparations, preferably aqueous compositions or water, against bacteria of the *Burkholderia cepacia* complex.

The present invention also relates to a process for treating products that may be contaminated by bacteria of the Bcc complex, in particular for treating i) water or an aqueous composition, ii) equipment, that may be industrial or domestic, such as industrial or domestic food, cosmetic or pharmaceutical preparation tanks, iii) the solvents used in industrial or domestic preparations, and iv) industrial or domestic anhydrous compositions, comprising at least one step of bringing into contact one or more compounds of formula (I) as defined above, in particular bringing into contact with i) water or aqueous compositions, ii) equipment, that may be industrial or domestic, preferably for cosmetic, pharmaceutical or food purposes, iii) solvents used in industrial or domestic preparations, and iv) industrial or domestic anhydrous compositions.

More particularly, the present invention relates to a process for treating water or an aqueous composition, comprising at least one step of bringing a sample of water or of an aqueous composition to be treated or of a stream of water to be treated, that is continuous or batchwise (batch-type), said water to be treated being chosen from domestic or industrial waters, waters from aquatic media, swimming pool/spa waters, and waters from air-conditioning systems, into contact with one or more compounds of formula (I) as defined above.

The present invention also relates to the treatment of waters contained in food, cosmetic or pharmaceutical products, preferably cosmetic products, against bacteria of the *Burkholderia cepacia* complex.

The present invention also relates to the treatment of *"anhydrous"* products or compositions, i.e. having an amount of water of less than 5%, preferably less than or equal to 3%, even more particularly less than or equal to 1%, in particular free of water, against bacteria of the *Burkholderia cepacia* complex. These anhydrous products may be food, cosmetic or pharmaceutical products, preferably cosmetic products.

The present invention also relates to the treatment of aqueous or anhydrous non-food, cosmetic or pharmaceutical products against bacteria of the *Burkholderia cepacia* complex.

**Definitions**

The expression *"treatment of a product or treatment of products"* is intended to mean a treatment, of the product(s), against bacteria of the *Burkholderia cepacia* complex, consisting in adding a substance to a sample of product(s) to be treated, continuously or batchwise (batch-type), for the purpose either of preventing the contamination of said product(s) by bacteria of the *Burkholderia cepacia* complex or of partially or totally decontaminating said product(s) to be treated, with respect to bacteria of the *Burkholderia cepacia* complex.

The term *"water treatment"* is intended to mean a continuous or batchwise (batch-type) treatment against bacteria of the *Burkholderia cepacia* complex, which consists in adding a substance to a sample of water to be treated or to a stream of water to be treated for the purpose either of preventing the contamination of the water by bacteria of the *Burkholderia cepacia* complex or of partially or totally decontaminating said water to be treated, with respect to bacteria of the *Burkholderia cepacia* complex.

Preferably, the treatment of water and of the aqueous compositions performed in the context of the present invention consists in adding, continuously or batchwise, a substance (a compound of formula (I) as defined above and in particular EZ) to a sample of water to be treated or to a stream of water to be treated in order to partially or totally decontaminate said water and aqueous compositions to be treated, with respect to a contaminating agent.

The contaminating agent according to the invention comprises bacteria of the *Burkholderia cepacia* complex.

The term *"waters from aquatic media"* is intended to mean the waters of lakes, tributary rivers, pools, mainstem

rivers, sea or ocean bathing areas, underground waters such as well waters and groundwaters, and aquarium waters.

The term *"aqueous composition"* is intended to mean a composition which comprises at least 5% of water, in particular between 5% and 99.9%, more particularly between 10% and 90% of water, preferentially between 20% 80% of water, more preferentially between 30% and 70% of water, and optionally comprises one or more organic solvents, in particular those defined below, i.e. organic solvents having Hansen solubility space parameters such that: $14.5 < \delta a < 30$ and $15 < \delta d < 22$. The aqueous composition is more particularly aqueousalcoholic, i.e. it comprises, in addition to the water, at least one ($C_2$-$C_6$)alkanol such as ethanol or a polyhydroxy($C_2$-$C_6$)alkanol such as glycerol.

Said *"aqueous"* composition can also comprise one or more fatty substances, in particular liquid at ambient temperature and atmospheric pressure, such as oils, in particular natural oils. The aqueous composition can comprise other ingredients conventionally used in foods, cosmetics or pharmaceuticals, such as those chosen from anionic, nonionic, cationic or zwitterionic surfactants, fatty substances such as oils, which are natural or synthetic, or derived from hydrocarbons, natural or non-natural, anionic, nonionic, cationic or zwitterionic, associative or non-associative thickening or non-thickening polymers, dyes and pigments.

The term *"anhydrous"* composition is intended to mean a composition which comprises an amount of water of less than 5%, preferably less than or equal to 3%, even more particularly less than or equal to 1%, in particular which is free of water.

The anhydrous composition can comprise other ingredients conventionally used in foods, cosmetics or pharmaceuticals, such as those chosen from i) anionic, nonionic, cationic or zwitterionic surfactants, ii) fatty substances, in particular oils, which are natural or synthetic, or derived from hydrocarbons, iii) natural or non-natural, anionic, nonionic, cationic or zwitterionic, associative or non-associative thickening or non-thickening polymers, iv) dyes, v) pigments, vi) fillers and vii) their mixture.

Said anhydrous composition can also comprise one or more fatty substances, in particular liquid at ambient temperature and atmospheric pressure, such as oils, in particular natural oils.

For the purposes of the present invention, the *"domestic or industrial waters"* comprise waste waters before they have been treated in a purification plant, waters undergoing treatment in a purification plant, waters before they have been treated in a drinking water plant, waters undergoing treatment in a drinking water plant, and also waters circulating in potable or non-potable urban networks, for instance waters circulating in pipes.

The term *"biofilm"* is intended to mean a deposit of microorganisms at the surface of a device in contact with water or solvents, this deposit being formed in particular by adsorption of microorganisms at the surface of said device. As device which comes into contact with the water or solvents, mention may for example be made of: pipes, in particular water pipes, cooling towers, etc.

The term *"industrial or domestic equipment"* is intended to mean any industrial or domestic tools or machines which help to create or to prepare, treat, convert or store natural or synthetic chemical products, biochemical products or biological products. Said equipment may be adjusted to the pressure, i.e. to a pressure below or above atmospheric pressure, preferably to a pressure greater than 1 atm, and to a high or low temperature, i.e. to a temperature above or below ambient temperature 25°C, preferably to a temperature above 30°C. In particular, said equipment is partly constituted of metal, glass, ceramic, and/or plastic such as PVC, or rubber. Preferably, the *equipment* of the invention is industrial. Preferentially, the *equipment* of the invention is constituted in part (which may be tanks or reactors, pipework, etc.) of metal such as aluminum, iron, copper, zinc, steel, stainless steel, cast iron, more preferentially of steel or stainless steel.

The term *"tank"* is intended to mean any domestic or industrial container for the preparation, treatment, conversion or storage of natural or synthetic chemical products, biochemical products or biological products. Said container may be adjusted to the pressure, i.e. to a pressure above or below atmospheric pressure, preferably to a pressure greater than 1 atm, and to a high or low temperature, i.e. to a temperature above or below ambient temperature 25°C, preferably to a temperature above 30°C. In particular, said container is constituted of metal, glass, ceramic, and/or plastic such as; the tank is preferably made of metal. Preferably, the tank in the invention is an industrial tank. Preferentially, the tank of the invention is made of metal such as aluminum, iron, copper, zinc, steel, stainless steel, cast iron, more preferentially of steel or stainless steel.

## Detailed description of the invention

*Use*

The compound of the following formula (I) and also the organic or mineral acid or base salts thereof, or solvates thereof such as hydrates:

[Chem. 1]

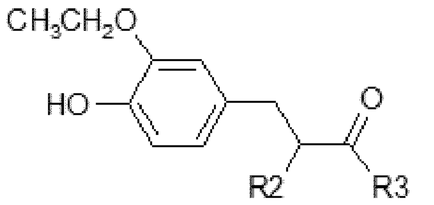

(I)

formula (I) wherein:

- R2 represents a hydrogen atom or a methyl or ethyl radical;
- R3 represents a linear $C_1$-$C_{12}$ alkyl radical (saturated), optionally substituted with a hydroxyl group; or a linear $C_2$-$C_{12}$ alkenyl radical (C=C unsaturated), optionally substituted with a hydroxyl group.

Preferably, the compounds correspond to formula (I), wherein:

- R2 is chosen from a hydrogen atom and a $CH_3$ group; even better still, R2 represents a hydrogen atom; and/or
- R3 represents (i) a $C_1$-$C_{10}$ alkyl radical; (ii) a $C_2$-$C_{10}$ alkenyl radical, in particular a -CH=CH-R4 radical with R4 representing a linear $C_1$-$C_6$ alkyl radical; or else (iii) a hydroxyalkyl radical having the structure -$CH_2$-CH(OH)-R5 with R5 representing a linear $C_1$-$C_{10}$, preferably $C_4$-$C_{10}$, alkyl radical.

A mixture of compounds of formula (I) can be used.
Mention may particularly be made of the following compounds of formula (I):

[Chem. 2]

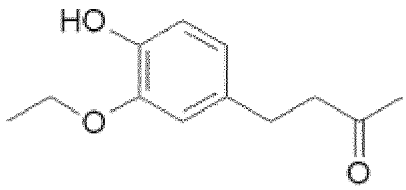

[Chem. 3]

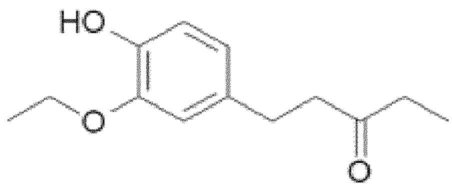

[Chem. 4]

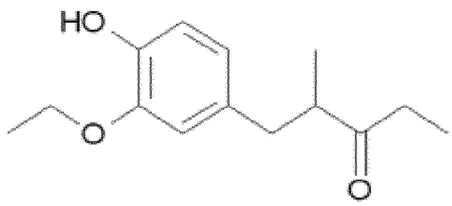

The compounds of formula (I) can be readily prepared by those skilled in the art on the basis of their general knowledge. Mention may be made especially of the following bibliographic references: J. Asian Natural Products Research, 2006, 8(8), 683-688; Helv. Chimica Acta, 2006, 89(3), 483-495; Chem. Pharm. Bull., 2006, 54(3), 377-379; and Bioorg. Med. Chem. Lett., 2004, 14(5), 1287-1289.
They may thus be prepared from ethylvanillin, in the manner described in the published patent application WO

2011/039445.

In one preferred embodiment of the present invention, use is made of 4-(3-ethoxy-4-hydroxyphenyl)butan-2-one having the following formula:

[Chem. 2]

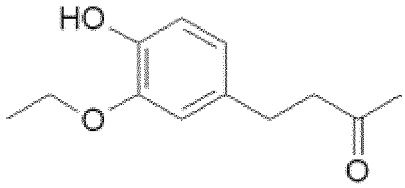

The compounds of formula (I) can be used in combination with at least one active agent chosen from anionic, cationic, nonionic or zwitterionic surfactants, descaling agents, coagulants, and mixtures thereof.

In one preferred embodiment, the compounds of formula (I) are used in combination with an effective amount of at least one organic solvent having solubility parameters according to the Hansen solubility space such that: $14.5 < \delta a < 30$ and $15 < \delta d < 22$.

Advantageously, the organic solvents used in the context of the present invention can be chosen from those described in the published patent application WO 2012/130953.

The total solubility parameter δ according to the Hansen solubility space is defined in the article "Solubility parameter values" by Eric A. Grulke in the book "Polymer Handbook", 3rd Edition, Chapter VII, pages 519-559, by the relationship:

[Math. 1]

$$\delta = (\delta^2 = \delta_d^2 + \delta_p^2 + \delta_h^2)^{1/2}$$

wherein

- δd characterizes the London dispersion forces derived from the formation of dipoles induced during molecular impacts,
- δp characterizes the Debye interaction forces between permanent dipoles,
- δh characterizes the specific interaction forces (such as hydrogen bonding, acid/base, donor/acceptor, etc.). The definition of solvents in the Hansen three-dimensional solubility space is described in the article by C.M. Hansen: "The three dimensional solubility parameters" J. Paint Technol. 39, 105 (1967).

The parameter δa is defined by the following relationship:

[Math. 2]

$$\delta_a^2 = \delta_p^2 + \delta_h^2 = \delta^2 - \delta_d^2$$

The parameters δd, δp, δh s and δa are expressed in (J/cm3)1/2.

Preferably, the organic solvent has solubility parameters such that $14.5 < \delta a < 28$ and $15 < \delta d < 20$.

The organic solvent used according to the invention can be chosen from ethanol (δa = 20.20; δd = 15.10), 1,2-propylene glycol (δa = 25.00; δd = 16.00), 1,3-propanediol (δa = 26.32; δd = 18.00), PEG-8 (polyethylene glycol containing 8 ethylene glycol units) (δa = 14.80; δd = 17.90), propylene carbonate (δa = 18.46; δd = 20.00), dipropylene glycol (δa = 19.48; δd = 16.20), 1,2-hexylene glycol (δa = 19.20; δd = 16.40), PEG-4 (δa = 18.60; δd = 18.00).

Preferably, the organic solvent is chosen from ethanol, 1,2-propylene glycol, 1,3-propanediol, PEG-8 and propylene carbonate.

The solvent may be used in a content ranging from 0.05% to 10% by weight relative to the total weight of the water to be treated comprising bacteria of the *Burkholderia cepacia* complex Bcc, preferably ranging from 0.1% to 5% by weight and preferentially ranging from 0.1% to 2.5% by weight relative to the total weight of the water to be treated comprising bacteria of the *Burkholderia cepacia* complex Bcc.

The compounds of formula (I), alone or as a mixture, can be used in a proportion of at least 0.06% by weight, preferably at least 0.1% by weight, even better still at least 0.5% by weight relative to the total weight of water to be treated comprising bacteria of the *Burkholderia cepacia* complex Bcc.

In one preferred embodiment, the compounds of formula (I), alone or as a mixture, can be used in a proportion of at least 1% by weight relative to the total weight of water to be treated comprising bacteria of the *Burkholderia cepacia* complex. The compounds of formula (I), alone or as a mixture, can be used in a concentration ranging from 0.06% to 10% by weight, preferably from 0.06% to 10% by weight, even better still from 0.06% to 5% by weight relative to the total weight of water to be treated comprising bacteria of the *Burkholderia cepacia* complex. In one preferred embodiment, the compounds of formula (I), alone or as a mixture, can be used in a concentration ranging from 0.05% to 2%, preferably ranging from 0.1% to 1% by weight relative to the total weight of water to be treated comprising bacteria of the *Burkholderia cepacia* complex.

*Process for treating water against the of the Burkholderia cepacia complex*

Preferably, said step of bringing the water to be treated and the aqueous composition into contact with the compound of formula (I) can in particular be carried out by injection in liquid form of said compound, by passage through a filter or a filtering cartridge comprising said compound, or by administration in solid form of said compound notably in the form of granules, lozenges or pellets.

*Process for treating a product that may be contaminated by bacteria of the Burkholderia cepacia complex*

According to one particular embodiment of the invention, the process for treating - in particular for preserving - a product that may be soiled by bacteria of the *Burkholderia cepacia* complex comprises the step of incorporating one or more compounds of formula (I) as defined above, preferably EZ, during the phase of producing said product.

The incorporation can be carried out by introducing into the preparation tank, beforehand, one or more compounds of formula (I), preferably EZ, preferably at a temperature of less than or equal to 80°C, better still at ambient temperature (25°C), and particularly at atmospheric pressure, then introducing into said tank the product to be preserved, or the ingredients required to produce said product. According to one variant, each ingredient required for the production of the product was treated beforehand with one or more compounds of formula (I), in particular EZ, or else each ingredient is introduced as a mixture with one or more compounds of formula (I), in particular EZ. The product is then preserved and made available to the user in the presence of one or more compounds of formula (I), in particular EZ.

According to another particular embodiment of the invention, the process for preserving a product that may be soiled by bacteria of the *Burkholderia cepacia* complex comprises the step of incorporating one or more compounds of formula (I) as defined above, preferably EZ, after the phase of producing said product. The product, once produced, is brought into contact with one or more compounds of formula (I), in particular EZ, preferably at a temperature of less than or equal to 80°C, better still at ambient temperature (25°C), and particularly at atmospheric pressure, then the product is preserved and made available to the user in the presence of one or more compounds of formula (I), in particular EZ.

. According to yet another particular embodiment of the invention, the process for preserving a product that may be soiled by bacteria of the *Burkholderia cepacia* complex comprises the step of incorporating one or more compounds of formula (I) as defined above, preferably EZ, after the phase of producing said product. The product, once produced, is stored under an inert atmosphere, then once opened by the user it is brought into contact with one or more compounds of formula (I), in particular EZ, preferably at ambient (25°C), and at atmospheric pressure, then the product is preserved in the presence of one or more compounds of formula (I), in particular EZ.

Preferably, the product is a composition, particularly comprising a physiologically acceptable medium, more particularly a cosmetic or pharmaceutical composition.

According to another embodiment of the invention, the product of the preserving process is not cosmetic.

The expressions *"between... and..."* and *"ranging from... to...", "at least* ..." or *"at most"* should be understood as being limits inclusive, unless otherwise specified.

The examples and figures that follow are provided as illustrations which do not limit the field of the invention.

**EXAMPLES**

***Sensitivity test***

The minimum inhibitory concentration (MIC) and the minimum bactericidal concentration (MBC) of compounds of formula (I) of the invention, in particular EZ, were determined by standardized agar dilution and broth dilution tests, described by Rushton et al. Key role for efflux in the preservative susceptibility and adaptive resistance of Burkholderia cepacia complex bacteria. Antimicrobial Agents and Chemotherapy, 2013. 57(7): p. 2972-2980 using TSA/TSB. The culture was carried out at 30°C in order to be representative of the production and storage of industrial products. Preserving stock solutions (50% *w/v*) were prepared in DMSO (Sigma-Aldrich, United Kingdom) and volumes were added to the medium so as to achieve the test concentration. The final concentration of DMSO in the presence of the bacterium

was verified to be non-toxic (not exceeding 4% v/v) and was included as control condition in the tests in order to eliminate the effect on growth. The MIC is defined as the lowest concentration of preservative at which there is an 80% reduction in the liquid OD in culture (at 630 nm), or no visible growth of the organisms tested on an agar medium. The MBC was determined as being the lowest concentration for causing a 99% destruction rate, and for which the growth on the recovery medium (TSA) stopped. The preservatives were inactivated before the recovery and counting of the surviving organisms tested, by dilution in a neutralizing solution containing 1.5% v/v of Tween 80 containing 3% of lecithin, as described in the scientific publication by Rushton et al. (Rushton et al, ibid). The efficacy and toxicity of the neutralizing solution were evaluated before the experiment, as described in the scientific publication by Lear, J.C., et al. (J.C., et al., Chloroxylenol- and triclosan-tolerant bacteria from industrial sources - Susceptibility to antibiotics and other biocides. International Biodeterioration and Biodegradation, 2006. 57(1): p. 51-56).

**Example 1** - **Evaluation of the inhibitory activity of a compound of formula (I) with respect to** the *Burkholderia cepacia* complex

***Bacterial strains and culture conditions.***

A panel of 59 *Burkholderia* used to profile the sensitivity to the compounds of formula (I), in particular EZ, was established. The collection comprised 20 of the current groups of species of the *Burkholderia cepacia* complex (Bcc), reference strains for the panel of Bcc experimental strains (Mahenthiralingam, E., et al., Diagnostically and experimentally useful panel of strains from the Burkholderia cepacia complex. Journal of Clinical Microbiology, 2000. 38(2): p. 910-913) and 39 strains previously profiled for sensitivity to the preservative by Rushton et al. (Rushton, L., et al., Key role for efflux in the preservative susceptibility and adaptive resistance of Burkholderia cepacia complex bacteria. Antimicrobial Agents and Chemotherapy, 2013. 57(7): p. 2972-2980). The strains of the *Burkholderia* panel were isolated from strains of clinical (n = 28), environmental (n = 23) and industrial (n = 8) origin. The strains were cultured once at 30°C on tryptone soy (TSB/TSA) (Oxoid Ltd, United Kingdom). The strains were stored in TSB containing 8% v/v of dimethyl sulfoxide (DMSO) (Sigma-Aldrich, UK) at -80°C.

[Table 1]: S1. *Burkholderia* strains tested

| Name of species and strain | BCCM / LMG culture collection accession number (Other strain designations) | Isolation source code |
|---|---|---|
| *B. ambifaria* | | |
| AMMD [ESP a] | LMG 19182 [T] | ENV |
| BCC0267 [ESP a] | LMG 19467 | CLIN |
| BCC0338 [a] | LMG 17828 | ENV |
| *B. anthina* | | |
| BCC0635 [ESP a] | LMG 16670 | ENV |
| BCC0639 [ESP a] | LMG 20980 [T] | ENV |
| BCC0485 [ESP] | LMG21821 | CLIN |
| *B. arboris* | | |
| BCC0049 [a] | - | CLIN |
| BCC1306 [a] | - | ENVI |
| BCC1310 [a] | - | ENVI |
| *B. cenocepacia III-A* | | |
| BCC0018 [ESP a] | LMG 16659 | CLIN |
| J2315 [ESP] | LMG 16656 [T] | CLIN |
| *B. cenocepacia III-B* | | |
| HI2424 [a] | - | ENV |
| *B. cepacia* | | |
| BCC0001 [ESP a] | LMG 1222 [T] | ENV |
| BCC0002 [ESP a] | LMG 2161 | ENV |
| BCC0003 [ESP a] | LMG 18821 | CLIN |
| *B. contaminans* | | |
| SAR-1 [a] | LMG 23255 | CLIN |
| BCC1315 [a] | - | ENVI |
| BCC0339 | - | CLIN |
| *B. diffusa* | | |
| BCC0106 [a] | LMG 24266 | CLIN |
| BCC0169 [a] | (ATCC 29352) | ENV |
| AU1075 [a] | LMG 24065 | CLIN |

| Name of species and strain | BCCM / LMG culture collection accession number (Other strain designations) | Isolation source code |
|---|---|---|
| *B. dolosa* | | |
| BCC0161 | FC0380 | CLIN |
| AU0645 [ESP a] | LMG 18943 [T] | CLIN |
| AU3556 [a] | - | CLIN |
| *B. lata* | | |
| Strain 383 [a] | LMG 22485 [T] | ENV |
| BCC1296 [a] | - | ENVI |
| BCC1406 [a] | - | ENVI |
| *B. latens* | | |
| BCC1625 | LMG24064[T] | CLIN |
| BCC1626 | LMG24264 | CLIN |
| BCC1892 | LMG24265 | CLIN |
| *B. metallica* | | |
| BCC0095 [a] | - | CLIN |
| AU0553 [a] | LMG 24068 [T] | CLIN |
| *B. multivorans* | | |
| BCC0149 | | CLIN |
| ATCC17616 [ESP a] | LMG 17588 | ENV |
| BCC1560 [a] | - | ENVI |
| *B. pseudomultivorans* | | |
| BCC1894 | LMG 26883 | CLIN |
| BCC1191 [a] | D1443 | CLIN |
| *B. pyrrocinia* | | |
| BCC0171 [ESP a] | LMG 21822 | ENV |
| BCC0180 [T ESP a] | LMG 14191 [T] | ENV |
| BCC0476 [ESP a] | LMG 21823 | ENV |
| *B. seminalis* | | |
| BCC1627 [T] | LMG 24067 | CLIN |
| BCC1628 | LMG 19587 | ENV |
| BCC1893 | LMG 24272 | CLIN |

| Name of species and strain | BCCM / LMG culture collection accession number (Other strain designations) | Isolation source code |
|---|---|---|
| *B. stabilis* | | |
| BCC0023 [ESP a] | LMG 14294 [T] | CLIN |
| BCC0286 [a] | (ATCC 35254) | ENV |
| AU6735 [a] | - | CLIN |
| *B. stagnalis* | | |
| BCC1350 | HI3541 | ENV |
| BCC1887 [T] | LMG 28156 | ENV |
| BCC1896 | LMG 28157 | ENV |
| *B. territorii* | | |
| BC1888 [T] | LMG 28158 | ENV |
| BCC1897 | LMG 28159 | ENV |
| *B. ubonensis* | | |
| BCC1603 [a] | LMG 20358 | ENV |
| *B. vietnamiensis* | | |
| BCC0028 [ESP a] | LMG 16232 | CLIN |
| BCC0195 | LMG 22486,G4 | ENV |
| BCC1309 [a] | - | ENVI |
| *B. gladioli* | | |
| BCC1317 [a] | | ENVI |
| BCC0238 | MA4 | CLIN |
| BCC0507 | PF(#428) | CLIN |
| *B. plantarii* | | |
| BCC0777 | LMG 9035 | ENV |

BCCM/LMG, Belgian co-ordinated collections of micro-organisms, Ghent.
ATCC, American type culture collection BCC.
CLIN, clinical; ENV, environmental; ENVI, environmental industrial.
T Type strain.
ESP experimental panel of experimental *Burkholderia cepacia* complex strains

The compounds of formula (I), in particular EZ, are very efficacious against bacteria of the *Burkholderia cepacia*

complex Bcc. The compounds of the invention, in particular EZ, demonstrated excellent efficacy against a diversified panel of 57 *Burkholderia* strains representing species commonly encountered as contaminants.

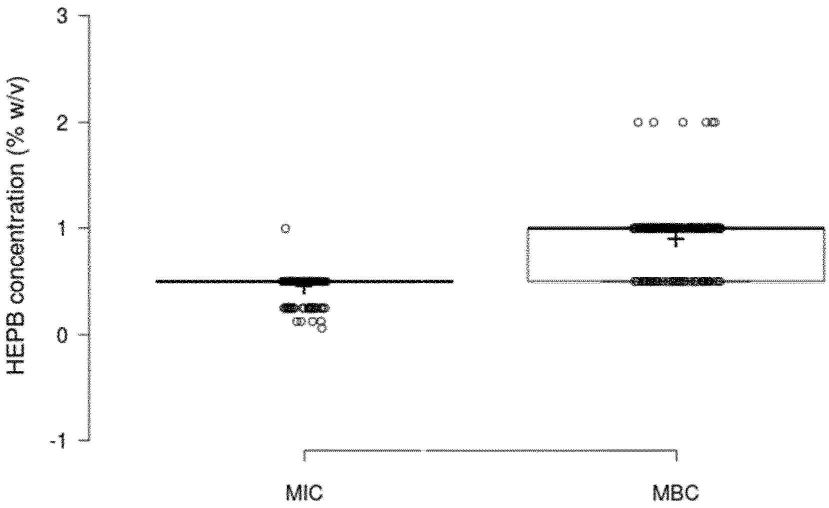


figure 1. Minimum inhibitory concentration (MIC) and minimum bactericidal concentration (MBC) of EZ for the panel of *Burkholderia* strains

In fig. 1,

- the center lines indicate the medians; the limits of the "box" indicate the 25th and 75th % determined by the free statistical software R; the Whiskers (i.e. method of representation of data in the box plot format - rectangle ranging from the first quartile to the third quartile cut by the median in purple on the graph above) extend over 1.5 times the interquartile range of the 25th and 75th %;
- the data points are represented in the form of open circles;
- the aberrant values are represented by dots;
- the crosses represent examples of means; and
- the width of the boxes is proportional to the square root of the size of the sample.

It appears that the compounds of formula (I) of the invention and in particular EZ act as a preservative with a high activity against bacteria of the *Burkholderia cepacia* complex leading in particular to a reduction in viability of several log after several hours (6 log in 4 hours).

All these experimental results show that the compounds of formula (I), in particular EZ, have a very significant inhibitory activity with respect to bacteria of the *Burkholderia cepacia* complex.

## Claims

1. The use of at least one compound of formula (I) and also the organic or mineral acid or base salts thereof, or solvates thereof such as hydrates:

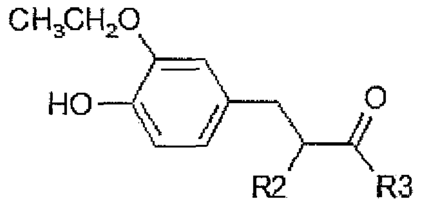 (I)

formula (I) wherein:

- R2 represents a hydrogen atom or a methyl or ethyl radical;
- R3 represents a linear $C_1$-$C_{12}$ alkyl radical (saturated), optionally substituted with a hydroxyl group; or a linear $C_2$-$C_{12}$ alkenyl radical (C=C unsaturated), optionally substituted with a hydroxyl group,

against bacteria of the *Burkholderia cepacia* complex (Bcc), in the treatment i) of water or an aqueous composition, ii) of equipment, that may be industrial or domestic, for food, cosmetic or pharmaceutical purposes, iii) of solvents used in industrial or domestic preparations, in particular aqueous compositions or water, and iv) of industrial or domestic anhydrous compositions, against bacteria of the *Burkholderia cepacia* complex.

**2.** The use as claimed in the preceding claim, for preventing the contamination of products that may be contaminated by bacteria of the *Bcc* complex, in particular the prevention of the contamination i) of water or an aqueous composition, ii) of equipment, that may be industrial or domestic, for food, cosmetic or pharmaceutical purposes, iii) of solvents used in industrial or domestic preparations, and iv) of industrial or domestic anhydrous compositions, in particular the prevention of the contamination of water and of aqueous compositions, by one or more microorganisms chosen from bacteria of the *Burkholderia* genus; said product, in particular said water or the water included in the aqueous composition, is particularly chosen from domestic or industrial waters, in particular those from industrial tanks, waters from a quartic media, swimming pool/spa waters, and air-conditioning system waters, but also cosmetic products.

**3.** The use as claimed in claim 1, for treating products that may be contaminated by bacteria of the *Bcc* complex, in particular the treatment of the contamination i) of water or an aqueous composition, ii) of equipment, that may be industrial or domestic, the food, cosmetic or pharmaceutical purposes, iii) of solvents used in industrial or domestic preparations, and iv) of industrial or domestic anhydrous compositions, in particular the treatment of water and of aqueous compositions, contaminated by one or more microorganisms chosen from bacteria of the *Burkholderia* genus, said product, in particular said water all the water included in the aqueous composition, is particularly chosen from domestic or industrial waters, in particular those from industrial tanks, waters from aquatic media, swimming pool/spa waters, and air-conditioning system waters, but also cosmetic products.

**4.** The use as claimed in any one of the preceding claims, wherein formula (I) is such that:

- R2 is chosen from hydrogen and methyl; even better still, R2 represents and/or
- R3 represents (i) a $C_1$-$C_{10}$ alkyl radical; (ii) a $C_2$-$C_{10}$ alkenyl radical, in particular a -CH=CH-R4 radical with R4 representing a linear $C_1$-$C_6$ alkyl radical; or else (iii) a hydroxyalkyl radical having the structure $-CH_2-CH(OH)-R5$ with R5 representing a linear $C_1$-$C_{10}$, preferably en $C_4$-$C_{10}$, alkyl radical.

**5.** The use as claimed in any one of the preceding claims, wherein the compound of formula (I) is chosen from the following compounds:

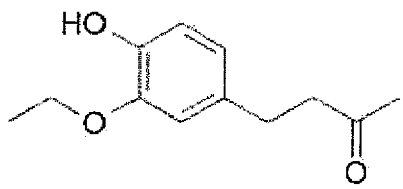

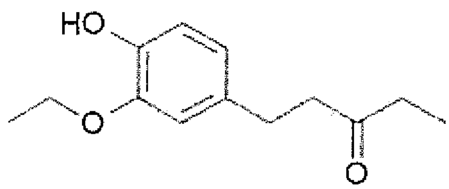

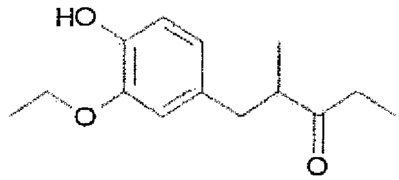

6. The use as claimed in any one of the preceding claims, wherein the compound of formula (I) is 4-(3-ethoxy-4-hydroxyphenyl)butan-2-one having the following formula:

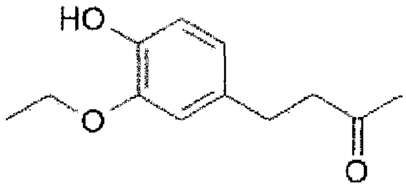

7. The use as claimed in any one of the preceding claims, wherein the compound of formula (I), alone or as a mixture, is used in a proportion of at least 0.06% by weight, preferably at least 0.1% by weight, even better still at least 0.5% by weight relative to the total weight of water or of aqueous composition to be treated.

8. A process for treating products, in particular water and aqueous compositions, making it possible i) either to prevent the contamination of said product, in particular water and aqueous compositions, by one or more microorganisms including bacteria of the *Burkholderia cepacia* complex, ii) or to treat said contaminated product, in particular water and aqueous compositions contaminated by one or more microorganisms including bacteria of the *Bcc* complex, comprising at least one step of bringing a sample of the product, in particular of water or of an aqueous composition to be treated or of a stream of water to be treated, that is continuous or batchwise, into contact with at least one compound of formula (I) as defined in any one of claims 1 to 6.

9. The process as claimed in the preceding claim, wherein the product, in particular water or the water included in aqueous compositions, is chosen from domestic or industrial products, particularly domestic or industrial waters, in particular from industrial tanks, waters from aquatic media, swimming pool/spa waters, and air-conditioning system waters, but also cosmetic products with the compound of formula (I) as defined in any one of claims 1 to 6.

10. The process as claimed in either one of claims 8 and 9, wherein the compound of formula (I), alone or as a mixture, is used in a proportion of at least 0.06% by weight, preferably at least 0.1% by weight, even better still at least 0.5% by weight relative to the total weight of water or of aqueous composition to be treated.

11. A process for treating, in particular preserving, a product that may be contaminated by bacteria of the *Burkholderia cepacia* complex, comprising a step of incorporating into said product, during the production thereof, or after the production thereof, and preferably before storage, one or more compounds of formula (I) as defined in any one of claims 1 to 6.

12. The process as claimed in the preceding claim, which comprises the step .of incorporating one or more compounds of formula (I) as defined in any one of claims 1 to 6, during the phase of producing said product.

13. The process as claimed in claim 11, wherein the incorporation is carried out by introducing into the preparation tank, beforehand, one or more compounds of formula (I), preferably at a temperature of less than or equal to 80°C, better still at ambient temperature (25°C), and particularly at atmospheric pressure, then by introducing, into said tank, the product to be treated and to be preserved, or the ingredients required to produce said product.

14. The process as claimed in claim 11 or 13, wherein each ingredient required for the production of the product has been treated beforehand with one or more compounds of formula (I), as defined in any one of claims 1 to 6, or else each ingredient is introduced as a mixture with one or more compounds of formula (I); then the product is preserved and made available to the user in the presence of one or more compounds of formula (I) as defined in any one of claims 1 to 6.

15. The process as claimed in claim 11 or 12, wherein the process comprises the step of incorporating one or more

compounds of formula (I) as defined in any one of claims 1 to 6, after the phase of producing said product, the product, once produced, is brought into contact with one or more compounds of formula (I), then the product is preserved and made available to the user in the presence of one or more compounds of formula (I),

**16.** An "anhydrous" composition, i.e. a composition having an amount of water of less than 5%, preferably less than or equal to 3%, even more particularly less than or equal to 1%, in particular free of water, comprising one or more compounds of formula (I) as defined in any one of claims 1 and 4 to 6; comprises one or more ingredients chosen from i) anionic, nonionic, cationic or zwitterionic surfactants, ii) fatty substances, in particular oils, which are natural or synthetic, or derived from hydrocarbons, iii) anionic, nonionic, cationic or zwitterionic, associative or non-associative thickening or non-thickening polymers, iv) dyes, v) pigments, and vi) their mixture.

**17.** A composition as claimed in the preceding claim, comprises one or more fatty substances, in particular liquid at ambient temperature and atmospheric pressure, such as oils, in particular natural oils.

## Patentansprüche

**1.** Verwendung wenigstens einer Verbindung der Formel (I) und auch der Organische-Säure-, Mineralsäure- oder Basensalze davon oder von Solvaten, wie z.B. Hydraten, davon:

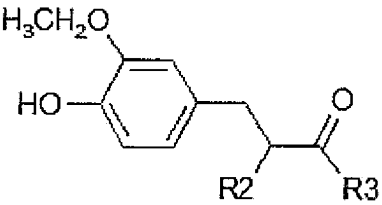

(I)

Formel (I) wobei:

- R2 ein Wasserstoffatom oder einen Methyl- oder Ethylrest darstellt;
- R3 einen linearen $C_1$-$C_{12}$-Alkylrest (gesättigt), gegebenenfalls substituiert mit einer Hydroxygruppe; oder einen linearen $C_2$-$C_{12}$-Alkenylrest (C=C-ungesättigt), gegebenenfalls substituiert mit einer Hydroxygruppe, darstellt,

gegen Bakterien des Burkholderia-cepacia-Komplexes (Bcc) bei der Behandlung i) von Wasser oder einer wässrigen Zusammensetzung, ii) von Ausrüstung, die industriell oder häuslich sein kann, für Lebensmittel-, kosmetische oder pharmazeutische Zwecke, iii) von Lösungsmitteln, die in industriellen oder häuslichen Zubereitungen, insbesondere wässrigen Zusammensetzungen oder Wasser, verwendet werden, und iv) von wasserfreien industriellen oder häuslichen Zusammensetzungen, gegen Bakterien des Burkholderia-cepacia-Komplexes.

**2.** Verwendung nach dem vorstehenden Anspruch zum Verhindern der Kontamination von Produkten, die durch Bakterien des Bcc-Komplexes kontaminiert werden können, insbesondere zum Verhindern der Kontamination i) von Wasser oder einer wässrigen Zusammensetzung, ii) von Ausrüstung, die industriell oder häuslich sein kann, für Lebensmittel-, kosmetische oder pharmazeutische Zwecke, iii) von Lösungsmitteln, die in industriellen oder häuslichen Zubereitungen verwendet werden, und iv) von wasserfreien industriellen oder häuslichen Zusammensetzungen, insbesondere Verhindern der Kontamination von Wasser und von wässrigen Zusammensetzungen durch einen oder mehrere Mikroorganismen ausgewählt aus Bakterien der Gattung Burkholderia; wobei das Produkt, insbesondere das Wasser oder das in der wässrigen Zusammensetzung enthaltene Wasser, insbesondere ausgewählt ist aus Haushalts- oder Industriewässern, insbesondere solchen aus Industrietanks, Wässern aus wässrigen Medien, Schwimmbad/Badewässern und Klimaanlagenwässern, aber auch kosmetischen Produkten.

**3.** Verwendung nach Anspruch 1 zur Behandlung von Produkten, die durch Bakterien des Bcc-Komplexes kontaminiert werden können, insbesondere zur Behandlung der Kontamination i) von Wasser oder einer wässrigen Zusammensetzung, ii) von Ausrüstung, die industriell oder häuslich sein kann, für Lebensmittel-, kosmetischen oder pharmazeutischen Zwecke, iii) von Lösungsmitteln, die in industriellen oder häuslichen Zubereitungen verwendet werden, und iv) von wasserfreien industriellen oder häuslichen Zusammensetzungen, insbesondere der Behandlung von Wasser und von wässrigen Zusammensetzungen, die durch einen oder mehrere Mikroorganismen ausgewählt aus Bakterien der Gattung Burkholderia kontaminiert sind, wobei das Produkt, insbesondere das Wasser, das gesamte in

der wässrigen Zusammensetzung enthaltene Wasser, insbesondere ausgewählt ist aus Haushalts- oder Industriewässern, insbesondere solchen aus Industrietanks, Wässern aus wässrigen Medien, Schwimmbad-/Badewässern und Klimaanlagenwässern, aber auch kosmetischen Produkten.

**4.** Verwendung nach einem der vorstehenden Ansprüche, wobei Formel (I) so ist, dass:

- R2 ausgewählt ist aus Wasserstoff und Methyl; noch besser R2 darstellt, und/oder
- R3 darstellt: (i) einen $C_1$-$C_{10}$-Alkylrest; (ii) einen C2-C10-Alkenylrest, insbesondere einen -CH=CH-R4-Rest, wobei R4 einen linearen $C_1$-$C_6$-Alkylrest darstellt; oder andernfalls (iii) einen Hydroxyalkylrest mit der Struktur - $CH_2$-CH(OH)-R5, wobei R5 einen linearen $C_1$-$C_{10}$-, vorzugsweise einen $C_4$-$C_{10}$-Alkylrest, darstellt.

**5.** Verwendung nach einem der vorstehenden Ansprüche, wobei die Verbindung der Formel (I) ausgewählt ist aus den folgenden Verbindungen:

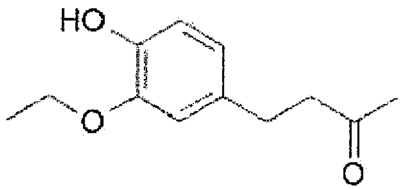

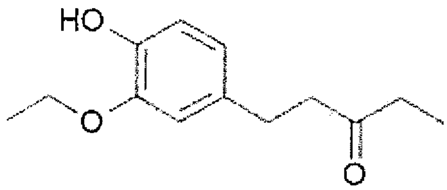

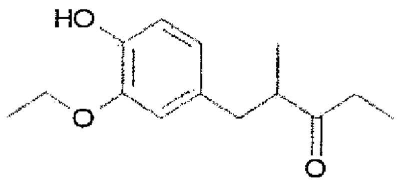

**6.** Verwendung nach einem der vorstehenden Ansprüche, wobei die Verbindung der Formel (I) 4-(3-Ethoxy-4-hydroxyphenyl)butan-2-on mit der folgenden Formel ist:

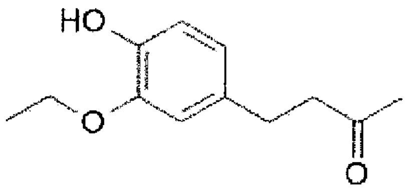

**7.** Verwendung nach einem der vorstehenden Ansprüche, wobei die Verbindung der Formel (I), allein oder in einem Gemisch, in einem Anteil von wenigstens 0,06 Gew.-%, vorzugsweise wenigstens 0,1 Gew.-%, noch besser wenigstens 0,5 Gew.-%, bezogen auf das Gesamtgewicht an Wasser oder wässriger Zusammensetzung, das/die behandelt werden soll, verwendet wird.

**8.** Verfahren zur Behandlung von Produkten, insbesondere Wasser und wässrigen Zusammensetzungen, das es ermöglicht i) entweder die Kontamination des Produkts, insbesondere Wasser und wässrigen Zusammensetzungen, durch einen oder mehrere Mikroorganismen, einschließlich Bakterien des Burkholderia-cepacia-Komplexes, zu verhindern, ii) oder das kontaminierte Produkt, insbesondere Wasser und wässrige Zusammensetzungen, die durch einen oder mehrere Mikroorganismen, einschließlich Bakterien des Bcc-Komplexes, kontaminiert sind, zu behandeln, umfassend wenigstens einen Schritt des Inkontaktbringens einer Probe des Produkts, insbesondere von Wasser oder einer wässrigen Zusammensetzung, das/die behandelt werden soll, oder von einem zu behandelnden Wasserstrom, der kontinuierlich oder diskontinuierlich ist, mit wenigstens einer Verbindung der Formel (I) nach einem der Ansprüche 1 bis 6.

**9.** Verfahren nach dem vorstehenden Anspruch, wobei das Produkt, insbesondere Wasser oder das in wässrigen Zusammensetzungen enthaltene Wasser, ausgewählt ist aus Haushalts- oder Industrieprodukten, insbesondere Haushalts- oder Industriewässern, insbesondere aus Industrietanks, Wässern aus wässrigen Medien, Schwimmbad-/Badewässern und Klimaanlagenwässern, aber auch kosmetischen Produkten, mit der Verbindung der Formel (I) nach einem der Ansprüche 1 bis 6.

**10.** Verfahren nach einem der Ansprüche 8 oder 9, wobei die Verbindung der Formel (I), allein oder in einem Gemisch, in einem Anteil von wenigstens 0,06 Gew.-%, vorzugsweise wenigstens 0,1 Gew.-%, noch besser wenigstens 0,5 Gew.-%, bezogen auf das Gesamtgewicht an Wasser oder wässriger Zusammensetzung, das/die behandelt werden soll, verwendet wird.

**11.** Verfahren zur Behandlung, insbesondere Konservierung, eines Produkts, das durch Bakterien des Burkholderia-cepacia-Komplexes kontaminiert werden kann, umfassend einen Schritt des Einverleibens einer oder mehrerer Verbindungen der Formel (I) nach einem der Ansprüche 1 bis 6 in das Produkt während dessen Herstellung oder nach dessen Herstellung und vorzugsweise vor der Lagerung.

**12.** Verfahren nach dem vorstehenden Anspruch, umfassend den Schritt des Einverleibens einer oder mehrerer Verbindungen der Formel (I) nach einem der Ansprüche 1 bis 6 während der Phase der Herstellung des Produkts.

**13.** Verfahren nach Anspruch 11, wobei das Einverleiben durch vorheriges Einführen einer oder mehrerer Verbindungen der Formel (I) in den Herstellungstank durchgeführt wird, vorzugsweise bei einer Temperatur von kleiner als oder gleich 80 °C, besser noch bei Umgebungstemperatur (25 °C), und insbesondere bei Atmosphärendruck, dann durch Einführen des zu behandelnden und zu konservierenden Produkts oder der zur Herstellung des Produkts erforderlichen Inhaltsstoffe in den Tank.

**14.** Verfahren nach Anspruch 11 oder 13, wobei jeder zur Herstellung des Produkts benötigte Inhaltsstoff zuvor mit einer oder mehreren Verbindungen der Formel (I) nach einem der Ansprüche 1 bis 6 behandelt worden ist, oder andernfalls jeder Inhaltsstoff als Gemisch mit einer oder mehreren Verbindungen der Formel (I) eingeführt wird; anschließend wird das Produkt konserviert und dem Anwender in Gegenwart einer oder mehrerer Verbindungen der Formel (I) nach einem der Ansprüche 1 bis 6 zur Verfügung gestellt.

**15.** Verfahren nach Anspruch 11 oder 12, wobei das Verfahren den Schritt des Einverleibens einer oder mehrerer Verbindungen der Formel (I) nach einem der Ansprüche 1 bis 6 nach der Phase der Herstellung des Produkts umfasst, wobei das Produkt, sobald hergestellt, mit einer oder mehreren Verbindungen der Formel (I) in Kontakt gebracht wird, anschließend wird das Produkt konserviert und dem Anwender in Gegenwart einer oder mehrerer Verbindungen der Formel (I) zur Verfügung gestellt.

**16.** "Wasserfreie" Zusammensetzung, d.h. Zusammensetzung mit einer Menge an Wasser von weniger als 5 %, vorzugsweise weniger als oder gleich 3 %, insbesondere weniger als oder gleich 1 %, insbesondere frei von Wasser, umfassend eine oder mehrere Verbindungen der Formel (I) nach einem der Ansprüche 1 und 4 bis 6; umfassend einen oder mehrere Inhaltsstoffe ausgewählt aus i) anionischen, nichtionischen, kationischen oder zwitterionischen Tensiden, ii) Fettstoffen, insbesondere Ölen, die natürlich oder synthetisch oder von Kohlenwasserstoffen abgeleitet sind, iii) anionischen, nichtionischen, kationischen oder zwitterionischen, assoziativen oder nicht assoziativen, verdickenden oder nicht verdickenden Polymeren, iv) Farbstoffen, v) Pigmenten und vi) deren Gemisch.

**17.** Zusammensetzung nach dem vorstehenden Anspruch, umfassend einen oder mehrere Fettstoffe, insbesondere bei Umgebungstemperatur und Atmosphärendruck flüssig, wie z.B. Öle, insbesondere natürliche Öle.

## Revendications

**1.** Utilisation d'au moins un composé de formule (I) ainsi que ses sels d'acide ou de base organique ou minéral(e), ou ses solvates tels que les hydrates :

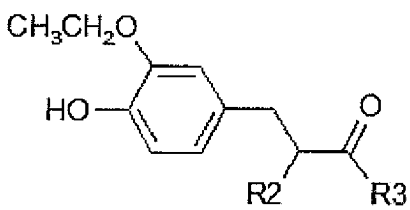

(I)

formule (I) dans laquelle :

- R2 représente un atome d'hydrogène ou un radical méthyle ou éthyle ;
- R3 représente un radical alkyle (saturé) linéaire en $C_1$-$C_{12}$, éventuellement substitué par un groupe hydroxyle ; ou bien un radical alcényle (insaturé C=C) linéaire en $C_2$-$C_{12}$, éventuellement substitué par un groupe hydroxyle,

contre des bactéries du complexe Burkholderia cepacia (Bcc), dans le traitement i) de l'eau ou d'une composition aqueuse, ii) d'équipements, qui peuvent être industriels ou domestiques, pour un usage alimentaire, cosmétique ou pharmaceutique, iii) de solvants utilisés dans des préparations industrielles ou domestiques, en particulier des compositions aqueuses ou l'eau, et iv) de compositions anhydres industrielles ou domestiques, contre des bactéries du complexe Burkholderia cepacia.

**2.** Utilisation selon la revendication précédente, pour empêcher la contamination de produits qui peuvent être contaminés par des bactéries du complexe Bcc, en particulier la prévention de la contamination i) de l'eau ou d'une composition aqueuse, ii) d'équipements, qui peuvent être industriels ou domestiques, pour un usage alimentaire, cosmétique ou pharmaceutique, iii) de solvants utilisés dans des préparations industrielles ou domestiques, et iv) de compositions anhydres industrielles ou domestiques, en particulier la prévention de la contamination de l'eau et de compositions aqueuses, par un ou plusieurs microorganismes choisis parmi les bactéries du genre Burkholderia ; ledit produit, en particulier ladite eau ou l'eau comprise dans la composition aqueuse, est particulièrement choisi parmi les eaux domestiques ou industrielles, en particulier celles des réservoirs industriels, des eaux de milieux aquatiques, des eaux de piscine/spa et des eaux de système d'air conditionné, mais aussi des produits cosmétiques.

**3.** Utilisation selon la revendication 1 pour le traitement de produits qui peuvent être contaminés par des bactéries du complexe Bcc, en particulier le traitement de la contamination i) de l'eau ou d'une composition aqueuse, ii) d'équipements, qui peuvent être industriels ou domestiques, pour un usage alimentaire, cosmétique ou pharmaceutique, iii) de solvants utilisés dans des préparations industrielles ou domestiques, et iv) de compositions anhydres industrielles ou domestiques, en particulier le traitement de l'eau et de compositions aqueuses, contaminées par un ou plusieurs microorganismes choisis parmi les bactéries du genre Burkholderia, ledit produit, en particulier ladite eau, toute l'eau comprise dans la composition aqueuse, est particulièrement choisi parmi les eaux domestiques ou industrielles, en particulier celles des réservoir industriels, des eaux de milieux aquatiques, des eaux de piscine/spa, des eaux de système d'air conditionné, mais aussi des produits cosmétiques.

**4.** Utilisation selon l'une quelconque des revendications précédentes, dans laquelle la formule (I) est telle que :

- R2 est choisi parmi hydrogène et méthyle ; encore mieux, R2 représente et/ou
- R3 représente (i) un radical alkyle en $C_1$-$C_{10}$ ; (ii) un radical alcényle en C2-C10, en particulier un radical
- CH=CH-R4, R4 représentant un radical alkyle linéaire en $C_1$-$C_6$ ; ou bien (iii) un radical hydroxyalkyle ayant la structure -$CH_2$-CH(OH)-R5, R5 représentant un radical alkyle linéaire en $C_1$-$C_{10}$, de préférence en $C_4$-$C_{10}$.

**5.** Utilisation selon l'une quelconque des revendications précédentes, dans laquelle le composé de formule (I) est choisi parmi les composés suivants :

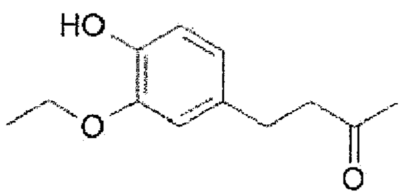

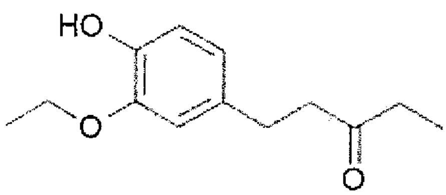

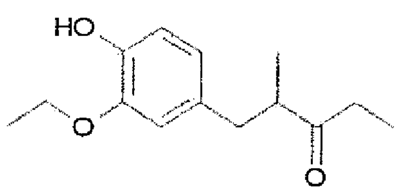

6. Utilisation selon l'une quelconque des revendications précédentes, dans laquelle le composé de formule (I) est la 4-(3-éthoxy-4-hydroxyphényl)butan-2-one de formule suivante :

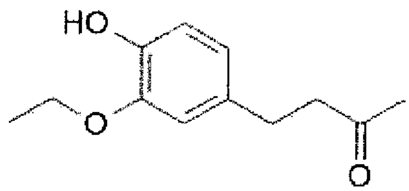

7. Utilisation selon l'une quelconque des revendications précédentes, dans laquelle le composé de formule (I), seul ou en mélange, est utilisé en une proportion d'au moins 0,06 % en poids, de préférence d'au moins 0,1 % en poids, encore mieux d'au moins 0,5 % en poids par rapport au poids total d'eau ou de composition aqueuse à traiter.

8. Procédé de traitement de produits, en particulier de l'eau et des compositions aqueuses permettant i) soit d'empêcher la contamination dudit produit, en particulier d'eau et de compositions aqueuses, par un ou plusieurs microorganismes comprenant des bactéries du complexe Burkholderia cepacia, ii) soit de traiter ledit produit contaminé, en particulier de l'eau et des compositions aqueuses contaminées par un ou plusieurs microorganismes comprenant des bactéries du complexe Bcc, comprenant au moins une étape de mise en contact d'un échantillon du produit, en particulier d'eau ou d'une composition aqueuse à traiter ou d'un flux d'eau à traiter, qui est continu ou discontinu, avec au moins un composé de formule (I) tel que défini dans l'une quelconque des revendications 1 à 6.

9. Procédé selon la revendication précédente, dans lequel le produit, en particulier l'eau ou l'eau comprise dans des compositions aqueuses, est choisi parmi les produits domestiques ou industriels, en particulier les eaux domestiques ou industrielles, en particulier parmi les réservoirs industriels, les eaux des milieux aquatiques, les eaux de piscine/spa et les eaux de système d'air conditionné, mais aussi les produits cosmétiques avec le composé de formule (I) tel que défini dans l'une quelconque des revendications 1 à 6.

10. Procédé selon l'une ou l'autre des revendications 8 et 9, dans lequel le composé de formule (I) seul ou en mélange, est utilisé en une proportion d'au moins 0,06 % en poids, de préférence d'au moins 0,1 % en poids, encore mieux d'au moins 0,5 % en poids par rapport au poids total d'eau ou de composition aqueuse à traiter.

11. Procédé de traitement, en particulier de conservation, d'un produit qui peut être contaminé par des bactéries du complexe Burkholderia cepacia, comprenant une étape d'incorporation dans ledit produit, au cours de sa production, ou après sa production, et de préférence avant stockage, d'un ou plusieurs composés de formule (I) tels que définis dans l'une quelconque des revendications 1 à 6.

12. Procédé selon la revendication précédente qui comprend l'étape d'incorporation d'un ou plusieurs composés de formule (I) tels que définis dans l'une quelconque des revendications 1 à 6, pendant la phase de production dudit produit.

13. Procédé selon la revendication 11, l'incorporation étant réalisée en introduisant dans la cuve de préparation au préalable un ou plusieurs composés de formule (I), de préférence à une température inférieure ou égale à 80 °C, encore mieux à température ambiante (25 °C), et en particulier à pression atmosphérique, puis en introduisant dans ladite cuve le produit à traiter et à conserver, ou les ingrédients nécessaires pour produire ledit produit.

14. Procédé selon la revendication 11 ou 13, dans lequel chaque ingrédient nécessaire à la production du produit a été préalablement traité par un ou plusieurs composés de formule (I), tels que définis dans l'une quelconque des revendications 1 à 6, ou alors chaque ingrédient est introduit en mélange avec un ou plusieurs composés de formule (I) ; puis le produit est conservé et mis à disposition de l'utilisateur en présence d'un ou plusieurs composés de formule (I) tels que définis dans l'une quelconque des revendications 1 à 6.

15. Procédé selon la revendication 11 ou 12, dans lequel le procédé comprend l'étape d'incorporation d'un ou plusieurs composés de formule (I) tels que définis dans l'une quelconque des revendications 1 à 6, après la phase de production dudit produit, le produit, une fois produit, est mis en contact avec un ou plusieurs composés de formule (I), puis le produit est conservé et mis à disposition de l'utilisateur en présence d'un ou plusieurs composés de formule (I).

16. Composition « anhydre », c'est-à-dire composition ayant une quantité d'eau inférieure à 5 %, de préférence inférieure

ou égale à 3 %, encore plus particulièrement inférieure ou égale à 1 %, en particulier exempte d'eau, comprenant un ou plusieurs composés de formule (I) tels que définis dans une quelconque des revendications 1 et 4 à 6 ; comprenant un ou plusieurs ingrédients choisis parmi i) les tensioactifs anioniques, non ioniques, cationiques ou zwittérioniques, ii) les substances grasses en particulier les huiles, qui sont naturelles ou synthétiques, ou dérivées d'hydrocarbures, iii) les polymères épaississants ou non épaississants associatifs ou non associatifs anioniques, non ioniques, cationiques ou zwittérioniques, iv) les colorants, v) les pigments, et vi) leur mélange.

**17.** Composition selon la revendication précédente comprenant une ou plusieurs substances grasses, en particulier liquides à température ambiante et pression atmosphérique, telles que des huiles, en particulier des huiles naturelles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011039445 A
- WO 2012130953 A


### Non-patent literature cited in the description


- **MARTIN M. RIEGER**. Kirk Othmer Encyclopedia. *Cosmetics*, 12 April 2000, https://doi.org/10.1002/0471238961.0315191318090507.a01
- **JIMENEZ, L.** Microbial diversity in pharmaceutical product recalls and environments.. *PDA Journal of Pharmaceutical Science and Technology*, 2007, vol. 61 (5), 383-399
- **RUSHTON, L.** *Rapid Alert System Weekly Notification Reports.*, 2005
- **RUSHTON, L. et al.** Key role for efflux in the preservative susceptibility and adaptive resistance of Burkholderia cepacia complex bacteria.. *Antimicrobial Agents and Chemotherapy*, 2013, vol. 57 (7), 2972-2980
- **MAHENTHIRALINGAM, E.** ; **A. BALDWIN** ; **C.G. DOWSON**. Burkholderia cepacia complex bacteria: Opportunistic pathogens with important natural biology.. *Journal of Applied Microbiology*, 2008, vol. 104 (6), 1539-1551
- **DEPOORTER, E. et al.** Burkholderia: an update on taxonomy and biotechnological potential as antibiotic producers.. *Applied Microbiology and Biotechnology*, 2016, vol. 100 (12), 5215-5229
- **WHITE, J. et al.** Culture-independent analysis of bacterial fuel contamination provides insight into the level of concordance with the standard industry practice of aerobic cultivation.. *Applied and Environmental Microbiology*, 2011, vol. 77 (13), 4527-4538
- **ÁLVAREZ-LERMA, F. et al.** Moisturizing body milk as a reservoir of Burkholderia cepacia: Outbreak of nosocomial infection in a multidisciplinary intensive care unit.. *Critical Care*, 2008, vol. 12 (1)
- **KUTTY, P.K. et al.** Multistate outbreak of Burkholderia cenocepacia colonization and infection associated with the use of intrinsically contaminated alcohol-free mouthwash.. *Chest*, 2007, vol. 132 (6), 1825-1831
- **TORBECK, L. et al.** Burkholderia cepacia: This decision is overdue.. *PDA Journal of Pharmaceutical Science and Technology*, 2011, vol. 65 (5), 535-543
- *J. Asian Natural Products Research*, 2006, vol. 8 (8), 683-688
- *Helv. Chimica Acta*, 2006, vol. 89 (3), 483-495
- *Chem. Pharm. Bull.*, 2006, vol. 54 (3), 377-379
- *Bioorg. Med. Chem. Lett.*, 2004, vol. 14 (5), 1287-1289
- Polymer Handbook. **ERIC A. GRULKE**. Solubility parameter values, 519-559
- **C.M. HANSEN**. The three dimensional solubility parameters. *J. Paint Technol.*, 1967, vol. 39, 105
- **RUSHTON et al.** Key role for efflux in the preservative susceptibility and adaptive resistance of Burkholderia cepacia complex bacteria.. *Antimicrobial Agents and Chemotherapy*, 2013, vol. 57 (7), 2972-2980
- **J.C. et al.** Chloroxylenol- and triclosan-tolerant bacteria from industrial sources - Susceptibility to antibiotics and other biocides.. *International Biodeterioration and Biodegradation*, 2006, vol. 57 (1), 51-56
- **MAHENTHIRALINGAM, E. et al.** Diagnostically and experimentally useful panel of strains from the Burkholderia cepacia complex.. *Journal of Clinical Microbiology*, 2000, vol. 38 (2), 910-913